# EUROPEAN PATENT SPECIFICATION

(11) **EP 3 546 952 B1**
(45) Date of publication and mention of the grant of the patent: **06.03.2024**
(21) Application number: 18164036.8
(22) Date of filing: 26.03.2018
(51) Int. Cl.: G01N 35/04

(54) **METHOD FOR UNSEALING AN OPENING OF A LABORATORY SAMPLE CONTAINER, METHOD FOR HANDLING A LABORATORY SAMPLE CONTAINER, LABORATORY APPARATUS AND LABORATORY AUTOMATION SYSTEM**
VERFAHREN ZUR ENTSIEGELUNG EINER ÖFFNUNG EINES LABORPROBENBEHÄLTERS, VERFAHREN ZUR HANDHABUNG EINES LABORPROBENBEHÄLTERS, LABORVORRICHTUNG UND LABORAUTOMATIONSSYSTEM
PROCÉDÉ DE DESCELLEMENT D'UNE OUVERTURE D'UN RÉCIPIENT D'ÉCHANTILLONS DE LABORATOIRE, PROCÉDÉ DE MANIPULATION D'UN RÉCIPIENT D'ÉCHANTILLONS DE LABORATOIRE, APPAREIL DE LABORATOIRE ET SYSTÈME D'AUTOMATISATION DE LABORATOIRE

(43) Date of publication of application: 02.10.2019
(73) Proprietor: Roche Diagnostics GmbH, 68305 Mannheim (DE); F. Hoffmann-La Roche AG, 4070 Basel (CH)
(72) Inventor: Mueller, Daniel, 6343 Rotkreuz (CH)
(74) Representative: Patentanwälte Ruff, Wilhelm, Beier, Dauster & Partner mbB

(56) References cited:
- EP-A1- 1 944 345
- WO-A1-2010/027283
- JP-A- 2013 108 797
- JP-A- 2017 026 381
- JP-B2- 2 941 464
- US-A1- 2002 041 828
- US-A1- 2007 134 131

## Description

### TECHNICAL FIELD AND PRIOR ART

The invention relates to a method for unsealing an opening of a laboratory sample container containing a sample, a method for handling a laboratory sample container containing a sample comprising such a method, a laboratory apparatus for unsealing an opening of a laboratory sample container containing a sample and a laboratory automation system comprising such a laboratory apparatus.

In laboratories some types of laboratory stations or instruments, respectively, and/or analysis may require laboratory sample containers such as sample tubes to be open for processing, in particular pretreating and/or analyzing, samples contained by the laboratory sample containers.

JP 2941464 B2 discloses a peeling apparatus which pulls apart a film-form cover from a sample tube with which an upper edge opening was covered by the film-form cover which has a tongue piece part extended outward.

US 2002/0041828 A1 discloses an analyzer system for lid-covered sample containers.

EP 1 944 345 A1 discloses a thermal-release double-coated pressure-sensitive adhesive tape or sheet.

US 2007/0134131 A1 discloses a sample distribution apparatus/system.

WO 2010/027283 A1 discloses a sample collection device suitable for low-volume extraction.

JP 2017 02381 A discloses a specimen sampling tube seal removal device removing a seal film from a specimen collection pipe with a film that includes a collected specimen and in which the seal film is firmly fixed to an opening. The specimen sampling tube seal removal device includes: a high frequency coil forming a magnetic field space; holding means configured to hold the opening of the specimen collection pipe with a film in the magnetic field space; and seal film bonding means of bonding the seal film and then lifting it.

### SUMMARY OF THE INVENTION

It is the object of the invention to provide a method for unsealing an opening of a laboratory sample container in an improved manner than in the prior art. It is a further object of the invention to provide a method for handling a laboratory sample container comprising such a method, a laboratory apparatus for unsealing an opening of a laboratory sample container and a laboratory automation system comprising such a laboratory apparatus.

These objects are solved by a method according to claim 1, a method according to claim 4, a laboratory apparatus according to claim 8 and a laboratory automation system according to claim 9. Preferred embodiments are defined in the dependent claims.

The invention relates to a method for unsealing, in particular automatically unsealing, or opening an opening or a mouth of a laboratory sample container containing or comprising a sample. The opening is sealed or closed by a closure attached or adhered to the laboratory sample container by an adhesive. An adhesive strength or a peel adhesion of the adhesive is lowerable or reducible or decreasable, in particular from a first value to a second value, by treatment, in particular external and/or physical treatment. The method comprises the steps: a) treating, in particular automatically treating, the adhesive, such that its adhesive strength is lowered, in particular from the first value to the second value. b) removing, in particular automatically removing, the closure from the laboratory sample container.

The method enables to unseal the opening of the laboratory sample container in a soft or gentle manner or way, in particular in comparison to unsealing the opening of the laboratory sample container without treating the adhesive and thereby without lowering the adhesive strength, but only removing, in particular pulling off, the closure from the laboratory sample container.

In particular the method may enable to reduce or to avoid a negative impact on the sample integrity. The method may enable to reduce or to prevent introducing a vibration or a shock to the laboratory sample container. Thereby, the method may enable to reduce or to prevent a spilling of the sample, in particular out of the laboratory sample container. Thereby, contamination or cross-contamination of the laboratory sample container and/or the sample and/or other laboratory sample containers and/or other samples and/or a laboratory apparatus and/or a laboratory automation system may be reduced or avoided.

Furthermore, the, in particular treating, method may enable that less or no adhesive residues are left, in particular on or at the laboratory sample container, after the unsealing. Thereby, a sealing, in particular a re-sealing, of the same opening of the same laboratory sample container may be facilitated, in particular repeated opening-closing-cycles may be allowed.

Moreover, the, in particular removing, method may enable that less or no damage is introduced to the laboratory sample container. Thereby, a sealing, in particular a re-sealing, of the same opening of the same laboratory sample container may be facilitated, in particular repeated opening-closing-cycles may be allowed.

Further, the method may enable, that no complex mechanics may be needed for unsealing.

Furthermore, the method may enable a fast unsealing, in particular 5 seconds or less per laboratory sample container.

In particular step b) may be performed simultaneously with step a) and/or after step a). In particular the closure may be left on or at the laboratory sample container or its opening during or even until after the treating of the adhesive or the lowering of its adhesive strength, respectively. Treating the adhesive or lowering the adhesive strength, respectively, may be denoted as releasing the closure. In particular then the closure may be, in particular easily, removed from the laboratory sample container, since the adhesive strength is lowered or the closure is released, respectively. Removing from the laboratory sample container may be denoted as taking up or off or peeling off from the laboratory sample container. Unsealing may be denoted as desealing.

The sample may be a blood sample, a serum sample, a plasma sample, a urine sample, a CFS sample, a body sample, a water sample, a chemical sample, a quality control (QC) material or a calibrator. In particular the sample may be a liquid.

The laboratory sample container may be designed as a tube and may have the opening at an upper, top and/or face end. In particular the laboratory sample container may be adapted to contain only one sample. Alternatively or additionally the laboratory sample container may be designed as a multi-well-plate and may have the opening at an upper end. In particular the laboratory sample container may be adapted to contain a plurality of samples, in particular separated samples. Furthermore, the laboratory sample container may be made of glass or transparent and/or translucent and/or opaque plastic, in particular polypropylene (PP) and/or polystyrene (PS) and/or polyethylene terephthalate (PET), or any other, in particular somewhat, solid material. The opening may be defined by an end of a wall and/or a circumference of the laboratory sample container.

The closure may comprise rubber and/or plastic and/or metal or may completely consist of rubber and/or plastic and/or metal. In particular a closure material may be polyvinyl chloride (PVC), polyolefin (PO), polyethylene terephthalate (PET) and/or aluminum. In particular the closure may comprise a sample-repellent property, in particular a liquid-tight property. The closure may enable a protection against evaporation, contamination and/or reaction of the sample and/or any other impact that might influence the sample, in particular influence pretreating and/or analyzing. Additionally or alternatively the closure may be embodied as a foil, in particular a flexible foil, or film or tape or as a lid, in particular as a rigid lid, or a plug, respectively. A thickness of the closure may be in the micrometer (µm) range, in particular in a range from 20 µm to 400 µm, in particular in a range from 50 µm to 300 µm, in particular in a range from 100 µm to 230 µm, in particular 100 µm.

The closure, in particular a surface of the closure, may be provided with the adhesive.

The second value, in particular after the treatment or treating, respectively, may be lower than the first value, in particular before the treatment or treating, respectively. In particular the adhesive may have a high enough adhesive strength or a high enough peel adhesion, respectively, before the treating, in particular for sealing or closing of the opening of the laboratory sample container, but which may be significantly reduced upon treating. In particular the adhesive strength, in particular its first value, before the treating may be in a range from 1000 Millinewton (mN) per 25 Millimeter (mm) to at least 50000 mN/25 mm, in particular in a range from 10000 mN/25 mm to 30000 mN/25 mm, in particular in a range from 15000 mN/25 mm to 25000 mN/25 mm. The, in particular lowered, adhesive strength, in particular its second value, after the treating may be in a range from 0 mN/25 mm to 1000 mN/25 mm, in particular in a range from 50 mN/25 mm to 800 mN/25 mm, in particular in a range from 100 mN/25 mm to 600 mN/25 mm. In particular the adhesive strength or its value, respectively, may be measured on stainless steel as an adherent with a removing or peeling angle of 180 degrees (°) and a removing or peeling speed of 300 mm/minute. Additionally or alternatively a thickness of the adhesive may be in the micrometer (µm) range, in particular in a range from 10 µm to 300 µm, in particular in a range from 30 µm to 200 µm, in particular in a range from 75 µm to 130 µm, in particular 130 µm. The adhesive may be denoted as glue.

In particular a thickness of the closure plus the adhesive may be in a range from 30 µm to 700 µm, in particular in a range from 80 µm to 500 µm, in particular in a range from 175 µm to 360 µm, in particular 230 µm.

The adhesive strength is lowerable by radiation treatment, in particular by ultraviolet (UV) radiation, and/or by heat treatment, in particular by heat with a temperature of minimal 50 degrees Celsius (°C), in particular of minimal 120 °C, and in particular of maximal 200 °C. Step a) comprises: treating the adhesive by radiation, in particular by UV radiation, and/or by heat, in particular by heat with a temperature of minimal 50 °C, in particular of minimal 120 °C, and in particular of maximal 200 °C. In particular this may be no normal conditions for the sample or the laboratory sample container. Thereby, the adhesive may not be inadvertently treated. In particular the UV radiation may be UV-A radiation, in particular having a wavelength from 380 to 315 Nanometer (nm), in particular 365 nm. The UV-A radiation may have little or no negative impact on the Deoxyribonucleic acid (DNA), if present, of the sample. The UV radiation may have an intensity of 100 Milliwatt (mW) per square centimeter (cm²), in particular of 1000 mW/cm². The UV radiation may have a dosage from 100 Millijoule (mJ) per cm² to 700 mJ/cm², in particular 500 mJ/cm². Additionally or alternatively, the radiation may be laser radiation. In particular the heat treatment may be performed in maximal 60 seconds (s), in particular in maximal 30 s, in particular in maximal 10 s, in particular in maximal 5 s, and in particular in minimal 1 s. Thereby, a negative impact on the sample by the heat treatment may be reduced or avoided. In particular the laboratory sample container may comprise a low heat conductivity. Thereby, a heat or thermal transfer from the opening of the laboratory sample container to the contained sample, which typically does not have to completely fill the laboratory sample container to its opening, may be reduced or avoided. This may be denoted as local treatment. The adhesive may be denoted radiation-sensitive and/or heat-sensitive adhesive.

According to an embodiment of the invention step a) comprises: exposing, in particular automatically exposing, the radiation to the adhesive and/or the opening and/or a rim of the laboratory sample container, respectively, along a direction being different from, in particular perpendicular to, a line between the adhesive and the sample. Additionally or alternatively step a) comprises: focusing, in particular automatically focusing, the radiation on the adhesive or the opening or the rim, respectively, in particular by an automatically influence able phase plate. Thereby, a negative impact on the sample, which typically does not have to completely fill the laboratory sample container to its opening, by the radiation treatment is reduced or avoided. This may be denoted as local treatment. In particular the radiation may be exposed under an angle or from below.

According to an embodiment of the invention the adhesive comprises a reactive site to UV radiation. In particular the adhesive may be denoted as UV curable. Additionally or alternatively the adhesive may comprise an acrylic copolymer, a photopolymerization initiator, a curing agent and a UV curable oligomer, in particular with a backbone formed out of polyester, epoxy or urethane and with a functional group, in particular diacrylourethane and/or UV curable polyfunctional monomers. In particular such an adhesive material may crosslink by the UV radiation treating. Thereby, the adhesive strength may be lowered, in particular by generation of microvoids in between the adhesive and the laboratory sample container suggested by volume contraction as possible mechanism. Additionally or alternatively the adhesive comprises a foaming agent, in particular heat-expandable microspheres. In particular such an adhesive material may foam or expand, respectively, by the heat treating. Thereby, the adhesive strength may be lowered, in particular by reducing an adhesion area, in particular its value, in between the adhesive and the laboratory sample container.

Step b) comprises: attaching, in particular automatically attaching, a take-up to the closure by another adhesive. Another adhesive strength, in particular its value, of the another adhesive is such that another adhesive force, in particular its value, between the take-up and the attached closure is higher than an adhesive force, in particular its value, between the closure and the laboratory sample container by the lowered adhesive strength of the treated adhesive. Removing, in particular automatically removing, the take-up with the attached closure from the laboratory sample container. Thereby, the force required to remove or to peel off, respectively, the take-up from the closure is higher than the force required to remove or to peel off, respectively, the closure from the laboratory sample container, in particular after the adhesive strength is lowered. In particular the required force, in particular its value, may depend on the another adhesive strength of the another adhesive and/or on the lowered adhesive strength of the treated adhesive and/or on an adhesion area, in particular its value, in between the take-up and the closure and/or on an adhesion area, in particular its value, in between the closure and the laboratory sample container and/or on a material property and/or a geometry of the closure and/or on a material property and/or a geometry of the laboratory sample container. Additionally or alternatively the another adhesive may be of the same adhesive type as the adhesive or another. Additionally or alternatively the take-up, in particular a surface of the take-up, may be provided with the another adhesive.

Step b) comprises: removing the closure from the laboratory sample container by at least one roller receptacle and mover supplying the take-up by a roller tape and moving the roller tape with the take-up with the attached closure from the laboratory sample container.

According to an embodiment of the invention the method may comprise the step: pre-treating the laboratory sample container, in particular its opening, in particular after removing the closure and/or before sealing the opening of the laboratory sample container, in particular by attaching a closure to the laboratory sample container, in particular by an adhesive. Thereby, an adhesion, in particular an adhesion strength or force value, of the, in particular attached, closure on or to the laboratory sample container may be improved, in particular increased, in particular in case the laboratory sample container comprises or consists of a low-energy material, in particular polypropylene (PP). Thereby, a risk, that the seal may be untight, in particular liquid-untight may be reduced or avoided.

The invention further relates to a method for handling, in particular for automatically handling, a laboratory sample container containing a sample. The method comprises the steps: sealing, in particular automatically sealing, or closing an opening of the laboratory sample container by attaching or adhering a closure to the laboratory sample container by an adhesive. An adhesive strength of the adhesive is lowerable, in particular from a first value to a second value, by treatment. Unsealing, in particular automatically unsealing, the opening by a method as described above, in particular after the sealing.

The sealing enables to store or to archive, respectively, and in particular to cool, the sample contained by the laboratory sample container. In particular the sample may be stored or archived at maximal 5 °C, and in particular at minimal minus 80 °C, in particular at minimal minus 30 °C. Additionally or alternatively the sealing may enable to move or to ship the sample contained by the laboratory sample container. Further, additionally or alternatively the method may enable a fast sealing.

The sample, the laboratory sample container, the closure and/or the adhesive may be embodied as described above.

Additionally or alternatively, in particular before sealing, the laboratory sample container, in particular its opening, may be pre-treated, in particular as described above.

According to an embodiment of the invention the adhesive is pressure-sensitive. The sealing comprises: pressing, in particular automatically pressing, the closure against the laboratory sample container, in particular with the adhesive in between the closure and the laboratory sample container.

According to an embodiment of the invention the method comprises the steps: supplying, in particular automatically supplying, the closure by a roller tape. Moving, in particular automatically moving, the roller tape with the closure to the opening of the laboratory sample container for the sealing. In particular the closure may be pre-cut or partially pre-cut and available from the roller tape.

According to an embodiment of the invention, the sealing comprises: attaching, in particular automatically attaching, a closure blank, in particular the roller tape, if present, to the laboratory sample container. Cutting-out, in particular automatically cutting-out, the closure out of the, in particular attached, closure blank, in particular the roller tape, if present. In particular the closure blank may be supplied by a roller tape as described above. Additionally or alternatively the closure blank, in particular the roller tape, may be partially pre-cut or perforated, respectively. Additionally or alternatively the closure blank, in particular a surface of the closure blank, may be provided with the adhesive. The cutting-out may comprise punching or laser cutting.

The invention further relates to a laboratory apparatus or device for unsealing an opening of a laboratory sample container containing a sample. The opening is sealed by a closure attached to the laboratory sample container by an adhesive. An adhesive strength of the adhesive is lowerable by treatment. The laboratory apparatus comprises a treater and a remover. The treater is adapted to treat the adhesive, such that its adhesive strength is lowered. The remover is adapted to remove the closure from the laboratory sample container.

In particular the laboratory apparatus may be adapted to perform a method, in particular for unsealing the opening of the laboratory sample container, as described above. By means of the method according to the invention, the advantages of the method according to the invention, as discussed above, may be made applicable for the laboratory apparatus.

Furthermore, the laboratory apparatus may enable, that no complex mechanics may be needed for unsealing. Thereby, a maintenance effort of the laboratory apparatus may be low.

Moreover, the laboratory apparatus may enable a fast unsealing. Thereby, the laboratory apparatus may enable a high throughput, in particular of up to 1200 samples or containers, respectively, per hour.

Further, the laboratory apparatus may have a compact design, in particular a small foot-print.

Furthermore, the laboratory apparatus or its treater or its remover, respectively, may be compatible with a small space or pitch between laboratory sample containers on a rack.

The laboratory apparatus may be denoted as unsealer.

The adhesive strength is lowerable by radiation treatment, in particular by UV radiation, and/or by heat treatment, in particular by heat with a temperature of minimal 50 °C, in particular of minimal 120 °C, and in particular of maximal 200 °C. The treater is adapted to treat the adhesive by radiation, in particular by UV radiation, and/or by heat, in particular by heat with a temperature of minimal 50 °C, in particular of minimal 120 °C, and in particular of maximal 200 °C. The treater may be denoted as radiation treater, in particular UV radiation treater. In particular the treater may comprise a radiation source, in particular a UV radiation source. Additionally or alternatively the treater may comprise an optic, in particular for exposing the radiation to the adhesive along a direction being different from a line between the adhesive and the sample and/or for focusing the radiation on the adhesive. Additionally or alternatively the treater may be denoted as heat treater. In particular the treater may comprise a heat source.

The remover is adapted to attach a take-up to the closure by another adhesive. Another adhesive strength of the another adhesive is such that an adhesive force between the take-up and the attached closure is higher than an adhesive force between the closure and the laboratory sample container by the lowered adhesive strength of the treated adhesive. Furthermore the remover is adapted to remove the take-up with the attached closure from the laboratory sample container.

The remover comprises at least one roller receptacle and mover. The roller receptacle and mover is adapted to supply the take-up by a roller tape, in particular of a take-up-roll. Furthermore, the roller receptacle and mover is adapted to move the roller tape with the take-up with the attached closure from the laboratory sample container.

According to an embodiment of the invention the laboratory apparatus may comprise a pre-treater. The pre-treater may be adapted to pre-treat the laboratory sample container, in particular its opening, in particular after removing the closure and/or before sealing the opening of the laboratory sample container, in particular by attaching a closure to the laboratory sample container, in particular by an adhesive.

The invention further relates to a laboratory automation system for handling a laboratory sample container containing a sample. The laboratory automation system comprises a sealer and a laboratory apparatus as described above. The sealer is adapted to seal an opening of the laboratory sample container by attaching a closure to the laboratory sample container by an adhesive. An adhesive strength of the adhesive is lowerable by treatment.

In particular the laboratory automation system or its sealer, respectively, may be adapted to perform the method, in particular for handling the laboratory sample container, in particular for sealing the opening of the laboratory sample container, as described above. By means of the method according to the invention, the advantages of the method according to the invention, as discussed above, may be made applicable for the laboratory automation system.

Furthermore, by means of the laboratory apparatus according to the invention, the advantages of the laboratory apparatus according to the invention, as discussed above, may be made applicable for the laboratory automation system.

Moreover, the laboratory automation system or its sealer, respectively may enable a fast sealing. Thereby, the laboratory automation system or its sealer, respectively, may enable a high throughput, in particular of up to 1500 samples or containers, respectively, per hour.

Further, the laboratory automation system or its sealer, respectively, may have a compact design, in particular a small foot-print.

Furthermore, the laboratory automation system or its sealer, respectively, may be compatible with a small space or pitch between laboratory sample containers on a rack.

Moreover, the sealer may be adapted to press the closure against the laboratory sample container. In particular the sealer may comprise a pressure pad.

Additionally or alternatively the laboratory automation system may comprise a, in particular the, pre-treater. The pre-treater may be adapted to pre-treat the laboratory sample container, in particular its opening, in particular before sealing the laboratory sample container, in particular its opening,

According to an embodiment of the invention the sealer comprises at least one roller receptacle and mover, which in particular may be different from the at least one roller receptacle and mover, if present, of the remover. The roller receptacle and mover is adapted to supply the closure by a roller tape, in particular of a closure-supply-roll or a closure-blank-supply-roll, respectively. Furthermore, the roller receptacle and mover is adapted to move the roller tape with the closure to the opening of the laboratory sample container for the sealing. In particular the sealer may be adapted to attach a closure blank, in particular the roller tape, to the laboratory sample container. Furthermore, the sealer may be adapted to cut-out the closure out of the, in particular attached, closure blank, in particular the roller tape. The sealer may comprise an attacher and a cutter. In particular the cutter may comprise a cutting or pinch blade and/or a laser.

The laboratory apparatus or its treater and/or its remover, respectively, and/or the laboratory automation system or its sealer, respectively, may be denoted as a laboratory station/s. The laboratory automation system may comprise a number of laboratory stations. The number of laboratory stations may comprise pre-analytical, analytical and/or post-analytical laboratory stations. Pre-analytical laboratory stations may be adapted to perform any kind of preprocessing of samples and/or laboratory sample containers. Analytical laboratory stations may be adapted to use a sample or part of the sample and a reagent to generate a measurement signal, the measurement signal indicating if and in which concentration, if any, an analyte is existing. Post-analytical laboratory stations may be adapted to perform any kind of post-processing of samples and/or laboratory sample containers. The pre-analytical, analytical and/or post-analytical laboratory stations may comprise at least one of an aliquot station, a centrifugation station, an archiving station, a pipetting station, a sorting station, a tube type identification station, a sample quality determining station, an add-on buffer station, a liquid level detection station, a decapping/recapping station, a pushing station, a belt station, a conveying system station and/or a gripper station.

Additionally the laboratory apparatus and/or the laboratory automation system may comprise a control device for controlling the laboratory apparatus and/or the laboratory automation system. The control device may comprise or be an integrated circuit, a tablet computer, a smartphone or a computer.

The step pre-treating and/or the pre-treater, respectively, may be independent of the unsealing, the sealing, the laboratory apparatus, the laboratory automation system, the sealer and/or the adhesive, in particular which adhesive strength is lowerable by treatment, an independent subject matter. In other words: a method may comprise the step: pre-treating a laboratory sample container, in particular its opening, in order to enhance an attaching force of a closure of the sample container to the sample container. According to an embodiment of this method the method may further comprise the step: sealing the pre-treated opening of the pre-treated laboratory sample container by attaching a closure to the laboratory sample container, in particular by an adhesive, in particular which adhesive strength may be lowerable by treatment. In other words: a protectable or inventive, respectively, pre-treater may be adapted to pre-treat a laboratory sample container, in particular its opening. Furthermore, a laboratory automation system may comprise the pre-treater and a sealer, wherein the sealer may be adapted to seal the, in particular pre-treated, opening of the pre-treated laboratory sample container by attaching a closure to the laboratory sample container, in particular by an adhesive, in particular which adhesive strength may be lowerable by treatment.

Thereby, an adhesion, in particular an adhesion strength or force value, of the, in particular attached, closure on or to the laboratory sample container may be improved, in particular increased, in particular in case the laboratory sample container comprises or consists of a low-energy material, in particular polypropylene (PP). Thereby, a risk, that the seal may be untight, in particular liquid-untight may be reduced or avoided.

In particular pre-treating may comprise increasing a surface free energy, in particular its value, of the laboratory sample container, in particular of a rim of the opening. In particular the pre-treating may comprise pre-treating by UV radiation, in particular by UV C radiation, by flame, by corona, by plasma and/or by acid etching and/or by the use of a solvent based adhesion promoter. In other words: a surface of the laboratory sample container or its rim, respectively, may be functionalized, in particular by oxygen, in particular by ozone.

Additionally or alternatively pre-treating may comprise increasing an adhesion area, in particular its value, of the laboratory sample container, in particular of the rim of the opening. In particular the pre-treating may comprise melting and/or roughening the laboratory sample container or its rim, respectively.

Additionally or alternatively pre-treating may comprise cleaning the laboratory sample container, in particular the rim of the opening, in particular from adhesive residues and/or liquids and/or coatings, in particular anticoagulants.

### BRIEF DESCRIPTION OF THE DRAWINGS

In the following, an embodiment of the invention will be described in detail with reference to the drawings. Throughout the drawings, the same elements will be denoted by the same reference numerals.
- Fig. 1: schematically shows a method for handling a laboratory sample container comprising a method for unsealing an opening of the laboratory sample container according to the invention and a laboratory automation system for handling the laboratory sample container comprising a laboratory apparatus for unsealing the opening of the laboratory sample container,
- Fig. 2: schematically shows a step treating by radiation of the method according to the invention and a treater for treating by radiation of the laboratory apparatus according to the invention,
- Fig. 3: schematically shows a step treating by heat of the method according to the invention and a treater for treating by heat of the laboratory apparatus according to the invention,
- Fig. 4: schematically shows a step removing and a remover,
- Fig. 5: schematically shows a step sealing the opening of the laboratory sample container of the method according to the invention and a sealer for sealing the opening of the laboratory sample container of the laboratory automation system according to the invention,
- Fig. 6: schematically shows a roller tape with a closure,
- Fig. 7: schematically shows a step sealing the opening of the laboratory sample container comprising steps attaching a closure blank to the laboratory sample container and cutting-out a closure out of the closure blank of the method according to the invention and a sealer for sealing the opening of the laboratory sample container by attaching the closure blank to the laboratory sample container and cutting-out the closure out of the closure blank of the laboratory automation system according to the invention and
- Fig. 8: schematically shows a roller tape of Fig. 7, from which the closure of Fig. 7 is cut-out.

### DETAILED DESCRIPTION OF EMBODIMENTS OF THE INVENTION

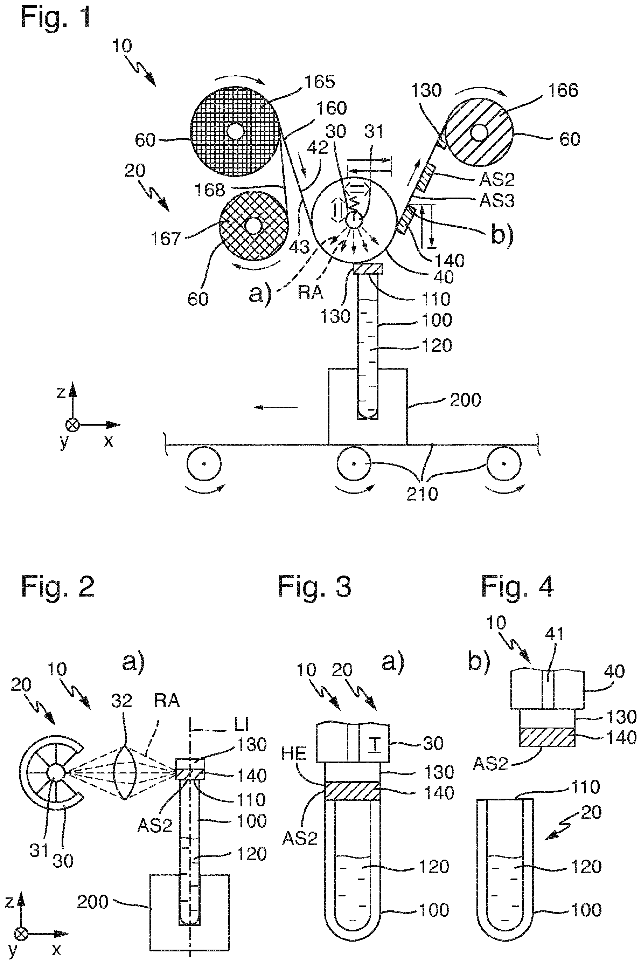

Fig. 1 to 4 show an inventive method for handling a laboratory sample container 100 containing a sample 120. The method comprises an inventive method for unsealing an opening 110 of the laboratory sample container 100 containing the sample 120. The opening 110 is sealed by a closure 130 attached to the laboratory sample container 100 by an adhesive 140. An adhesive strength AS1 of the adhesive 140 is lowerable AS2 by treatment, in particular from a first value AS1 to a second value AS2. The method comprises the steps: a) treating the adhesive 140, such that its adhesive strength AS1 is lowered AS2, in particular from the first value AS1 to the second value AS2. b) removing the closure 130 from the laboratory sample container 100.

Furthermore, Fig. 1 to 4 show an inventive laboratory automation system 10 for handling the laboratory sample container 100 containing the sample 120. The laboratory automation system 10 comprises an inventive laboratory apparatus 20 for unsealing the opening 110 of the laboratory sample container 100 containing the sample 120. The opening 110 is sealed by the closure 130 attached to the laboratory sample container 100 by the adhesive 140. The adhesive strength AS1 of the adhesive 140 is lowerable AS2 by treatment. The laboratory apparatus 20 comprises a treater 30 and a remover 40. The treater 30 is adapted to treat the adhesive 140, such that its adhesive strength AS1 is lowered AS2. The remover 40 is adapted to remove the closure 130 from the laboratory sample container 100.

The laboratory automation system 10 is adapted to perform the method, in particular for handling the laboratory sample container 100, as described above. The laboratory apparatus 20 is adapted to perform the method, in particular for unsealing the opening 110 of the laboratory sample container 100, as described above.

In detail the laboratory sample container 100 is designed as a tube. The, in particular upright, laboratory sample container 100 has the opening 110 at an upper end of a wall, as shown in Fig. 4. The laboratory sample container 100 contains the sample 120 in form of a liquid. The sample 120 does not completely fill the laboratory sample container 100 up to its opening 110. In other words: a surface of the sample 120 is significantly away from the opening 110.

As shown in Fig. 1 to 3, the opening 110 is sealed by the closure 130. In the shown embodiment the closure 130 is embodied as a foil. In alternative embodiments the closure may be embodied as a lid.

In the embodiment shown in Fig. 1 and 2 the adhesive 140 comprises a reactive site to UV radiation.

The adhesive strength AS1 of the adhesive 140 is lowerable AS2 by radiation treatment, in particular by UV radiation RA.

The treater 30 is adapted to treat the adhesive 140 by radiation RA, in particular by UV radiation. In particular the treater 30 comprises a radiation source 31, in particular a UV radiation source 31.

Step a) comprises: treating the adhesive 140 by radiation RA, in particular by UV radiation RA, in particular by the treater 30.

In detail step a) comprises: exposing the radiation RA to the adhesive 140 along a direction x being different from, in particular perpendicular to, a line LI between the adhesive 140 and the sample 120, as shown in Fig. 2. Additionally step a) comprises: focusing the radiation on the adhesive 140.

In particular the treater 30 comprises an optic 32, in particular for exposing the radiation RA to the adhesive 140 along the direction x being different from the line LI between the adhesive 140 and the sample 120 and for focusing the radiation RA on the adhesive 140.

In alternative embodiments step a) may comprise either exposing the radiation to the adhesive along a direction being different from a line between the adhesive and the sample or focusing the radiation on the adhesive. Additionally or alternatively in alternative embodiments the treater may comprise an optic, in particular either for exposing the radiation to the adhesive along a direction being different from a line between the adhesive and the sample or for focusing the radiation on the adhesive.

In the embodiment shown in Fig. 3 and 4 the adhesive 140 comprises a foaming agent, in particular heat-expandable microspheres.

The adhesive strength AS1 of the adhesive 140 is lowerable AS2 by heat treatment, in particular by heat HE with a temperature T of minimal 50 °C.

The treater 30 is adapted to treat the adhesive 140 by heat HE, in particular by heat with a temperature T of minimal 50 °C. In particular the treater 30 comprises a heat source.

Step a) comprises: treating the adhesive 140 by heat HE, in particular by heat HE with a temperature T of minimal 50 °C, in particular by the treater 30.

In detail the treater 30 is, in particular brought, in heat or thermal contact with the adhesive 140 for the heat treating. In particular the treater 30 is brought in direct contact with the closure 130, in Fig. 3 from the top and/or along the direction z. In alternative embodiments the adhesive may be treated by thermal radiation, in particular by infrared radiation, in particular by a laser.

In the shown embodiments the closure 130 is left on the laboratory sample container 100 until after the treating of the adhesive 140 or the lowering of its adhesive strength AS2, respectively. After the treating the, in particular released, closure 130 may be easily removed from the laboratory sample container 100.

In the embodiment shown in Fig. 1 the remover 40 is adapted to attach a take-up 42 to the closure 130 by another adhesive 43. Another adhesive strength AS3 of the another adhesive 43 is such that another adhesive force between the take-up 42 and the attached closure 130 is higher than an adhesive force between the closure 130 and the laboratory sample container 100 by the lowered adhesive strength AS2 of the treated adhesive 140. Furthermore the remover 40 is adapted to remove the take-up 42 with the attached closure 130 from the laboratory sample container 100.

Step b) comprises: attaching the take-up 42 to the closure 130 by the another adhesive 43, in particular by the remover 40. The another adhesive strength AS3 of the another adhesive 43 is such that the another adhesive force between the take-up 42 and the attached closure 130 is higher than the adhesive force between the closure 130 and the laboratory sample container 100 by the lowered adhesive strength AS2 of the treated adhesive 140. Removing the take-up 42 with the attached closure 130 from the laboratory sample container 100, in particular by the remover 40.

The take-up 42, in particular a surface of the take-up 42, is provided with the another adhesive 43.

In particular the another adhesive 43 is pressure-sensitive and the take-up 42 is attached to the closure 130 by pressing the take-up 42 against the closure 130, in Fig. 1 from the top and/or along the direction z as shown by arrows.

In detail the remover 40 comprises at least one roller receptacle and mover 60. The roller receptacle and mover 60 is adapted to supply the take-up 42 by a roller tape 160, in particular of a take-up-roll 165. Furthermore, the roller receptacle and mover 60 is adapted to move the roller tape 160 with the take-up 42 with the attached closure 130 from the laboratory sample container 100, in Fig. 1 from the left to the right and/or along the direction x, z.

In particular the remover 40 comprises a plurality of, in particular three, roller receptacles and movers 60. One roller receptacle and mover 60 is adapted to provide the take-up-roll 165 and to rotate the take-up-roll 165 and thereby the roller tape 160, as shown in Fig. 1 by arrows. This roller receptacle and mover 60 may be denoted as upstream-side roller. Another roller receptacle and mover 60 is adapted to provide a waste-roll 166 and to rotate the waste-roll 166 and thereby the roller tape 160. This roller receptacle and mover 60 may be denoted as downstream-side roller. Another roller receptacle and mover 60 is adapted to provide a release-liner-roll 167 and to rotate the release-liner-roll 167 and thereby a release liner roller tape 168.

The release liner roller tape 168 is adapted to protect the roller tape 160 and its take-up 42 and the another adhesive 43 before the unsealing and to be released from the roller tape 160 and its take-up 42 and the another adhesive 43 for the unsealing.

Furthermore, the release liner roller tape 168 comprises or consists of polyester.

Moreover, the laboratory automation system 10 or its laboratory apparatus 20, respectively, comprises a holder 200, in particular in form of a single holder for the single or only laboratory sample container 100. The holder 200 is adapted to hold the laboratory sample container 100 or its opening 110, respectively, aligned with respect to the treater 30 and/or the remover 40 for the unsealing, in particular in the upright position. In detail the holder 200 is adapted to hold, in particular to surround, the laboratory sample container 100 at, in particular a lower part, of a circumference of the laboratory sample container 100.

In alternative embodiments the laboratory automation system or its laboratory apparatus, respectively, may comprise two holders, three holders or more than three holders, in particular at least ten holders, in particular at least one-hundred holders, in particular at least one-thousand holders. Additionally or alternatively in alternative embodiments the laboratory automation system or its laboratory apparatus, respectively, may comprise at least one rack, in particular for five laboratory sample containers, wherein the rack may be adapted to hold the laboratory sample container/s aligned with respect to treater and/or the remover for the unsealing.

Further, the laboratory automation system 10 or its laboratory apparatus 20, respectively, comprises a movement unit 210. The movement unit 210 is adapted to move the laboratory sample container 100 or the holder 200 with the held laboratory sample container 100, respectively, to/from the treater 30 and/or the remover 40, in particular with the opening 110 below the treater 30 and/or the remover 40, in Fig. 1 from the right to the left and/or along the direction x.

In the shown embodiment the movement unit 210 is embodied as a conveyor belt. In alternative embodiments the movement unit may comprise or be a band and/or a laboratory sample container distribution system as described in EP 2 995 958 A1.

In alternative embodiments the laboratory automation system or its laboratory apparatus, respectively, may comprise either the holder or the movement unit.

In the embodiment shown in Fig. 4 the remover 40 comprises a sucker 41. The sucker 41 is adapted to suck the closure 130 from the laboratory sample container 100 by vacuum.

Step b) comprises: sucking the closure 130 from the laboratory sample container 100 by vacuum, in particular by the remover 40 or its sucker 41, respectively.

In particular the sucker 41 comprises a vacuum chuck and/or a vacuum source.

The embodiment shown in Fig. 3 and 4 may also comprise a holder and/or a movement unit, as shown in the embodiment of Fig. 1 and 2.

In the embodiment shown in Fig. 3 and 4 the treater 30, in particular in form of the heat treater, and the remover 40, in form of the sucker 41, are embodied as one-piece or combined, respectively. In alternative embodiments the treater and the remover may be embodied separate from each other.

In the embodiment shown in Fig. 1 the treater 30, in particular in form of the radiation treater, and the remover 40, in particular with the roller tape 160, the take-up 42 and the adhesive 43, are embodied as one-piece or combined, respectively. In detail the roller tape 160, the take-up 42 and the adhesive 43 are transparent for the radiation RA. In alternative embodiments the treater and the remover may be embodied separate from each other.

Furthermore, in alternative embodiments heat treating does not have to be combined with sucking by vacuum. In alternative embodiments heat treating may be combined with attaching a take-up and removing the take-up. Additionally or alternative in alternative embodiments radiation treating does not have to be combined with attaching a take-up and removing the take-up. In alternative embodiments radiation treating may be combined with sucking by vacuum. In particular the sucker may be transparent for the radiation.

The above described, in particular treating, method/s enable/s that less or no adhesive residues are left, in particular on the laboratory sample container 100, after the unsealing.

Furthermore, the above described, in particular removing, method/s enable/s that less or no damage is introduced to the laboratory sample container 100.

Thereby, a sealing, in particular a re-sealing, of the same opening 110 of the same laboratory sample container 100 is facilitated.

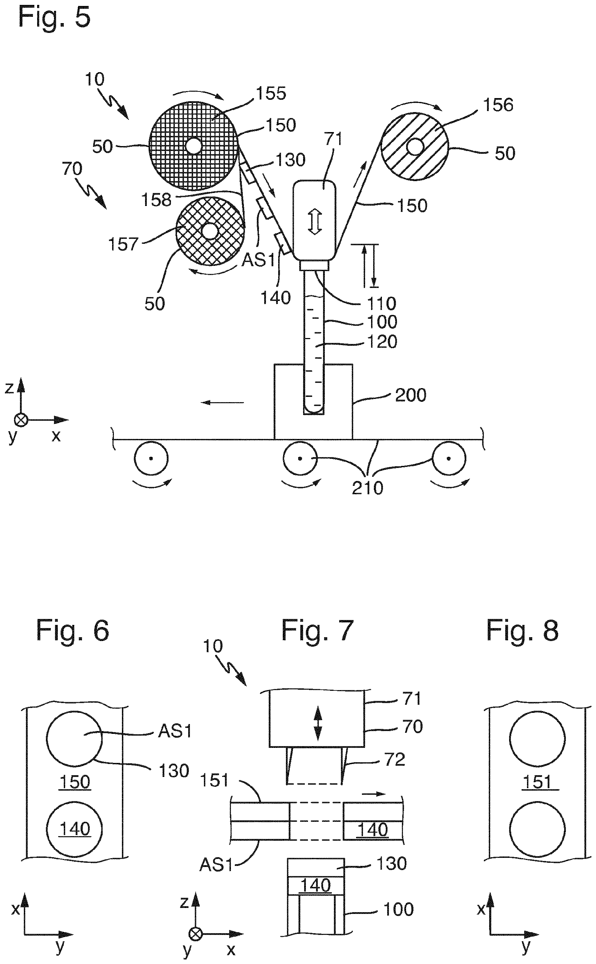

Furthermore, the method for handling a laboratory sample container 100 containing a sample 120 comprises the step: sealing the opening 110 of the laboratory sample container 100, in particular before the unsealing. The sealing comprises attaching a closure 130 to the laboratory sample container 100 by an adhesive 140, as shown in Fig. 5 to 8. An adhesive strength AS1 of the adhesive 140 is lowerable AS2 by treatment, in particular from a first value AS1 to a second value AS2.

Moreover, the laboratory automation system 10 for handling the laboratory sample container 100 containing the sample 120 comprises a sealer 70, as shown in Fig. 5 to 8. The sealer 70 is adapted to seal the opening 110 of the laboratory sample container 100 by attaching the closure 130 to the laboratory sample container 100 by the adhesive 140. The adhesive strength AS1 of the adhesive 140 is lowerable AS2 by treatment.

The laboratory automation system 10 or its sealer 70 is adapted to perform the method, in particular sealing the opening 110 of the laboratory sample container 100, as described above.

The closure 130, in particular a surface of the closure 130, is provided with the adhesive 140.

In detail the adhesive 140 is pressure-sensitive.

The sealer 70 is adapted to press the closure 130 against the laboratory sample container 100, in Fig. 5 and 7 from the top and/or along the direction z as shown by arrows. In particular the sealer 70 comprises a pressure pad 71.

The sealing comprises: pressing the closure 130 against the laboratory sample container 100, in particular with the adhesive 140 in between the closure 130 and the laboratory sample container 100, in particular by the sealer 70 or its pressure pad 71, respectively.

The sealer 70 comprises at least one roller receptacle and mover 50. The roller receptacle and mover 50 is adapted to supply the closure 130 by a roller tape 150, 151, in particular of a closure-supply-roll 155 or a closure-blank-supply-roll, respectively. Furthermore, the roller receptacle and mover 50 is adapted to move the roller tape 150, 151 with the closure 130 to the opening 110 of the laboratory sample container 100 for the sealing, in Fig. 5 and 7 from the left to the right and/or along the direction x, z.

The method comprises the steps: supplying the closure 130 by the roller tape 150, 151, in particular by the at least one roller receptacle and mover 50. Moving the roller tape 150, 151 with the closure 130 to the opening 110 of the laboratory sample container 100 for the sealing, in particular by the at least one roller receptacle and mover 50.

In particular the sealer 70 comprises a plurality of, in particular three, roller receptacles and movers 50. One roller receptacle and mover 50 is adapted to provide the closure-supply-roll 155 and/or the closure-blank-supply-roll and to rotate the closure-supply-roll 155 and/or the closure-blank-supply-roll and thereby the roller tape 150, 151, as shown in Fig. 5 and 7 by arrows. This roller receptacle and mover 50 may be denoted as upstream-side roller. Another roller receptacle and mover 50 is adapted to provide a carrier-roll and/or a waste-roll 156 and to rotate the carrier-roll and/or the waste-roll 156 and thereby the roller tape 150, 151. This roller receptacle and mover 50 may be denoted as downstream-side roller. Another roller receptacle and mover 50 is adapted to provide a release-liner-roll 157 and to rotate the release-liner-roll 157 and thereby a release liner roller tape 158.

The release liner roller tape 158 is adapted to protect the roller tape 150, 151, and in particular its closure 130, before the sealing and to be released from the roller tape 150,151, and in particular its closure 130, for the sealing.

Furthermore, the release liner roller tape 158 comprises or consists of polyester.

Moreover, the laboratory automation system 10 or its sealer 70, respectively, comprises a holder 200, in particular in form of a single holder for the single or only laboratory sample container 100. The holder 200 is adapted to hold the laboratory sample container 100 or its opening 110, respectively, aligned with respect to the sealer 70 for the sealing, in particular in the upright position.

In detail the holder 200 is or may be embodied as the holder 200 described above for the embodiment shown in Fig. 1.

Additionally or alternatively in alternative embodiments the laboratory automation system or its sealer, respectively, may comprise at least one rack, in particular for five laboratory sample containers, wherein the rack may be adapted to hold the laboratory sample container/s aligned with respect to the sealer for the sealing.

Further, the laboratory automation system 10 or its sealer 70, respectively, comprises a movement unit 210. The movement unit 210 is adapted to move the laboratory sample container 100 or the holder 200 with the held laboratory sample container 100, respectively, to/from the sealer 70, in particular with the opening 110 below the sealer, in Fig. 5 and 7 from the right to the left and/or along the direction x.

In detail the movement unit 210 is or may be embodied as the movement unit 210 described above for the embodiment shown in Fig. 1.

In alternative embodiments the laboratory automation system or its sealer, respectively, may comprise either the holder or the movement unit.

In the embodiment shown in Fig. 5 and 6 the closure 130 is pre-cut and available from the roller tape 150, in particular of the closure-supply-roll 155.

In the embodiment shown in Fig. 7 and 8 the sealer 70 is adapted to attach a closure blank 151, in particular the roller tape 151, to the laboratory sample container 100, in Fig. 7 from the top and/or along the direction z as shown by an arrow. Furthermore, the sealer 70 is adapted to cut-out the closure 130 out of the, in particular attached, closure blank 151, in particular the roller tape 151.

In particular the sealer 70 comprises an attacher 71, in particular in form of the pressure pad 71, and a cutter 72, in particular in form of a blade 72. In the embodiment shown in Fig. 7 and 8 the attacher 71 and the cutter 72 are embodied as one-piece or combined, respectively. In alternative embodiments the attacher and the cutter may be embodied separate from each other.

The sealing comprises: attaching the closure blank 151, in particular the roller tape 151, to the laboratory sample container 100, in particular by the sealer 70 or its attacher 71, respectively. Cutting-out the closure 130 out of the, in particular attached, closure blank 151, in particular the roller tape 151, in particular by the sealer 70 or its cutter 72, respectively.

The closure blank 151, in particular a surface of the closure blank 151, is provided with the adhesive 140.

In detail the closure blank 151 may be supplied by the roller tape 151, in particular as described above for the embodiment shown in Fig. 5.

In particular a part of the roller tape 151, which covers the opening 110 of the laboratory sample container 100, is cut-out from the roller tape 151 by the cutter 72 in form of the cylindrical blade.

After the cutting-out the roller tape 151 is still connected, as shown in Fig. 8, in particular in between the closure-blank-supply-roll and the waste-roll.

The laboratory apparatus 20 or its treater 30 and/or its remover 40, respectively, and/or the laboratory automation system 10 or its sealer 70, respectively, may be denoted as a laboratory station/s. Beyond that the laboratory automation system may comprise a number of other laboratory stations. The movement unit 210 is or may be adapted to move the laboratory sample container 100 between the laboratory stations.

As the shown and above discussed embodiments reveal, the invention provides a method for unsealing an opening of a laboratory sample container in an improved manner than in the prior art, in particular in a soft manner. Furthermore, the invention provides a method for handling a laboratory sample container comprising such a method, a laboratory apparatus for unsealing an opening of a laboratory sample container and a laboratory automation system comprising such a laboratory apparatus.

## Claims

1. Method for unsealing an opening (110) of a laboratory sample container (100) containing a sample (120), wherein the opening (110) is sealed by a closure (130) attached to the laboratory sample container (100) by an adhesive (140), wherein an adhesive strength (AS1) of the adhesive (140) is lowerable (AS2) by radiation treatment, in particular by UV radiation (RA), and/or by heat treatment, in particular by heat (HE) with a temperature (T) of minimal 50 °C, in particular of minimal 120 °C, the method comprising the steps:
- a) treating the adhesive (140), such that its adhesive strength (AS1) is lowered (AS2), step a) comprising: treating the adhesive (140) by radiation (RA), in particular by UV radiation (RA), and/or by heat (HE), in particular by heat (HE) with a temperature (T) of minimal 50 °C, in particular of minimal 120 °C, and
- b) removing the closure (130) from the laboratory sample container (100)
- the method being **characterized in that** step b) comprises: attaching a take-up (42) to the closure (130) by another adhesive (43), wherein another adhesive strength (AS3) of the another adhesive (43) is such that another adhesive force between the take-up (42) and the attached closure (130) is higher than an adhesive force between the closure (130) and the laboratory sample container (100) by the lowered adhesive strength (AS2) of the treated adhesive (140), and removing the take-up (42) with the attached closure (130) from the laboratory sample container (100),
- step b) further comprising: removing the closure (130) from the laboratory sample container (100) by at least one roller receptacle and mover (60) supplying the take-up (42) by a roller tape (160) and moving the roller tape (160) with the take-up (42) with the attached closure (130) from the laboratory sample container (100).

2. Method according to claim 1, step a) comprising:
- exposing the radiation (RA) to the adhesive (140) along a direction (x) being different from a line (LI) between the adhesive (140) and the sample (120) and/or
- focusing the radiation (RA) on the adhesive (140).

3. Method according to claim 1 or 2,
- wherein the adhesive (140) comprises a reactive site to UV radiation (RA) and/or a foaming agent.

4. Method for handling a laboratory sample container (100) containing a sample (120), the method comprising the steps:
- sealing an opening (110) of the laboratory sample container (100) by attaching a closure (130) to the laboratory sample container (100) by an adhesive (140), wherein an adhesive strength (AS1) of the adhesive (140) is lowerable (AS2) by treatment, and
- unsealing the opening (110) by a method according to any one of the preceding claims.

5. Method according to claim 4,
- wherein the adhesive (140) is pressure-sensitive, and
- the sealing comprising: pressing the closure (130) against the laboratory sample container (100).

6. Method according to claim 4 or 5, comprising the steps:
- supplying the closure (130) by a roller tape (150, 151) and moving the roller tape (150, 151) with the closure (130) to the opening (110) of the laboratory sample container (100) for the sealing.

7. Method according to any one of claims 4 to 6, the sealing comprising:
- attaching a closure blank (151) to the laboratory sample container (100) and
- cutting-out the closure (130) out of the closure blank (151).

8. Laboratory apparatus (20) for unsealing an opening (110) of a laboratory sample container (100) containing a sample (120), wherein the opening (110) is sealed by a closure (130) attached to the laboratory sample container (100) by an adhesive (140), wherein an adhesive strength (AS1) of the adhesive (140) is lowerable (AS2) by
radiation treatment, in particular by UV radiation (RA), and/or by heat treatment, in particular by heat (HE) with a temperature (T) of minimal 50 °C, in particular of minimal 120 °C, the laboratory apparatus (20) comprising:
- a treater (30), wherein the treater (30) is adapted to treat the adhesive (140), such that its adhesive strength is lowered (AS2), wherein the treater (30) is adapted to treat the adhesive (140) by radiation (RA), in particular by UV radiation (RA), and/or by heat (HE), in particular by heat (HE) with a temperature (T) of minimal 50 °C, in particular of minimal 120 °C, and
- a remover (40), wherein the remover (40) is adapted to remove the closure (130) from the laboratory sample container (100),
- **characterized in that** the remover (40) is adapted to attach a take-up (42) to the closure (130) by another adhesive (43), wherein another adhesive strength (AS3) of the another adhesive (43) is such that an adhesive force between the take-up (42) and the attached closure (130) is higher than an adhesive force between the closure (130) and the laboratory sample container (100) by the lowered adhesive strength (AS2) of the treated adhesive (140), and to remove the take-up (42) with the attached closure (130) from the laboratory sample container (100),
- the remover (40) comprising: at least one roller receptacle and mover (60), wherein the roller receptacle and mover (60) is adapted to supply the take-up (42) by a roller tape (160) and to move the roller tape (160) with the take-up (42) with the attached closure (130) from the laboratory sample container (100).

9. Laboratory automation system (10) for handling a laboratory sample container (100) containing a sample (120), the laboratory automation system (10) comprising:
- a sealer (70), wherein the sealer (70) is adapted to seal an opening (110) of the laboratory sample container (100) by attaching a closure (130) to the laboratory sample container (100) by an adhesive (140), wherein an adhesive strength (AS1) of the adhesive (140) is lowerable (AS2) by treatment, and
- a laboratory apparatus (20) according to claim 8.

10. Laboratory automation system (10) according to claim 9, the sealer (70) comprising:
- at least one roller receptacle and mover (50), wherein the roller receptacle and mover (50) is adapted to supply the closure (130) by a roller tape (150, 151) and to move the roller tape (150, 151) with the closure (130) to the opening (110) of the laboratory sample container (100) for the sealing.

## Patentansprüche

1. Verfahren zum Entsiegeln einer Öffnung (110) eines Laborprobenbehälters (100), der eine Probe (120) enthält, wobei die Öffnung (110) mit einem Verschluss (130) versiegelt ist, der mit einem Haftmittel (140) an dem Laborprobenbehälter (100) angebracht ist, wobei eine Haftfestigkeit (AS1) des Haftmittels (140) durch Strahlenbehandlung, insbesondere durch UV-Strahlung (RA), und/oder durch Wärmebehandlung, insbesondere durch Hitze (HE) mit einer Temperatur (T) von minimal 50 °C, insbesondere von minimal 120 °C, verringert werden kann (AS2), wobei das Verfahren die folgenden Schritte umfasst:
- a) Behandeln des Haftmittels (140), sodass seine Haftfestigkeit (AS1) verringert wird (AS2), wobei Schritt a) Folgendes umfasst: Behandeln des Haftmittels (140) durch Strahlung (RA), insbesondere durch UV-Strahlung (RA), und/oder durch Hitze (HE), insbesondere durch Hitze (HE) mit einer Temperatur (T) von minimal 50 °C, insbesondere von minimal 120 °C, und
- b) Entfernen des Verschlusses (130) von dem Laborprobenbehälter (100);
- wobei das Verfahren **dadurch gekennzeichnet ist, dass** Schritt b) Folgendes umfasst: Anbringen eines Aufnehmers (42) an dem Verschluss (130) mit einem anderen Haftmittel (43), wobei eine andere Haftfestigkeit (AS3) des anderen Haftmittels (43) derart ist, dass eine andere Haftkraft zwischen dem Aufnehmer (42) und dem angebrachten Verschluss (130) um die verringerte Haftfestigkeit (AS2) des behandelten Haftmittels (140) höher ist als eine Haftkraft zwischen dem Verschluss (130) und dem Laborprobenbehälter (100), und Entfernen des Aufnehmers (42) mit dem angebrachten Verschluss (130) von dem Laborprobenbehälter (100),
- wobei Schritt b) ferner Folgendes umfasst: Entfernen des Verschlusses (130) von dem Laborprobenbehälter (100) mit mindestens einer Rollenaufnahme- und -bewegungseinrichtung (60), wodurch der Aufnehmer (42) mit einem Rollenband (160) versehen und das Rollenband (160) mit dem Aufnehmer (42) mit dem angebrachten Verschluss (130) von dem Laborprobenbehälter (100) wegbewegt wird.

2. Verfahren nach Anspruch 1, wobei Schritt a) Folgendes umfasst:
- Bestrahlen (RA) des Haftmittels (140) entlang einer Richtung (x), die verschieden ist von einer Linie (LI) zwischen dem Haftmittel (140) und der Probe (120) und/oder
- Richten der Strahlung (RA) auf das Haftmittel (140).

3. Verfahren nach Anspruch 1 oder 2,
- wobei das Haftmittel (140) eine für UV-Strahlung (RA) empfängliche Stelle und/oder einen Schaumbildner umfasst.

4. Verfahren zum Handhaben eines Laborprobenbehälters (100), der eine Probe (120) enthält, wobei das Verfahren die folgenden Schritte umfasst:
- Versiegeln einer Öffnung (110) des Laborprobenbehälters (100) durch Anbringen eines Verschlusses (130) an dem Laborprobenbehälter (100) mit einem Haftmittel (140), wobei eine Haftfestigkeit (AS1) des Haftmittels (140) mittels Behandlung verringert werden kann (AS2), und
- Entsiegeln der Öffnung (110) durch ein Verfahren nach einem der vorstehenden Ansprüche.

5. Verfahren nach Anspruch 4,
- wobei das Haftmittel (140) selbstklebend ist und
- das Versiegeln Folgendes umfasst: Drücken des Verschlusses (130) gegen den Laborprobenbehälter (100).

6. Verfahren nach Anspruch 4 oder 5, umfassend die folgenden Schritte:
- Versehen des Verschlusses (130) mit einem Rollenband (150, 151) und Bewegen des Rollenbandes (150, 151) mit dem Verschluss (130) zu der Öffnung (110) des Laborprobenbehälters (100) zum Versiegeln.

7. Verfahren nach einem der Ansprüche 4 bis 6, wobei das Versiegeln Folgendes umfasst:
- Anbringen eines Verschlussrohlings (151) an dem Laborprobenbehälter (100) und
- Ausschneiden des Verschlusses (130) aus dem Verschlussrohling (151).

8. Laborvorrichtung (20) zum Entsiegeln einer Öffnung (110) eines Laborprobenbehälters (100), der eine Probe (120) enthält, wobei die Öffnung (110) mit einem Verschluss (130) versiegelt ist, der mit einem Haftmittel (140) an dem Laborprobenbehälter (100) angebracht ist, wobei eine Haftfestigkeit (AS1) des Haftmittels (140) durch Strahlenbehandlung, insbesondere durch UV-Strahlung (RA), und/oder durch Wärmebehandlung, insbesondere durch Hitze (HE) mit einer Temperatur (T) von minimal 50 °C, insbesondere von minimal 120 °C, verringert werden kann (AS2), wobei die Laborvorrichtung (20) Folgendes umfasst:
- eine Behandlungseinrichtung (30), wobei die Behandlungseinrichtung (30) zum Behandeln des Haftmittels (140) ausgelegt ist, sodass seine Haftfestigkeit verringert wird (AS2), wobei die Behandlungseinrichtung (30) zum Behandeln des Haftmittels (140) durch Strahlung (RA), insbesondere durch UV-Strahlung (RA), und/oder durch Hitze (HE), insbesondere durch Hitze (HE) mit einer Temperatur (T) von minimal 50 °C, insbesondere von minimal 120 °C, ausgelegt ist, und
- eine Entfernungseinrichtung (40), wobei die Entfernungseinrichtung (40) zum Entfernen des Verschlusses (130) von dem Laborprobenbehälter (100) ausgelegt ist,
- **dadurch gekennzeichnet, dass** die Entfernungseinrichtung (40) dazu ausgelegt ist, einen Aufnehmer (42) an dem Verschluss (130) mit einem anderen Haftmittel (43) anzubringen, wobei eine andere Haftfestigkeit (AS3) des anderen Haftmittels (43) derart ist, dass eine Haftkraft zwischen dem Aufnehmer (42) und dem angebrachten Verschluss (130) um die verringerte Haftfestigkeit (AS2) des behandelten Haftmittels (140) höher ist als eine Haftkraft zwischen dem Verschluss (130) und dem Laborprobenbehälter (100), und den Aufnehmer (42) mit dem angebrachten Verschluss (130) von dem Laborprobenbehälter (100) zu entfernen,
- wobei die Entfernungseinrichtung (40) Folgendes umfasst: mindestens eine Rollenaufnahme- und -bewegungseinrichtung (60), wobei die Rollenaufnahme- und -bewegungseinrichtung (60) zum Versehen des Aufnehmers (42) mit einem Rollenband (160) und zum Wegbewegen des Rollenbandes (160) mit dem Aufnehmer (42) mit dem angebrachten Verschluss (130) von dem Laborprobenbehälter (100) ausgelegt ist.

9. Laborautomationssystem (10) zum Handhaben eines Laborprobenbehälters (100), der eine Probe (120) enthält, wobei das Laborautomationssystem (10) Folgendes umfasst:
- eine Versiegelungseinrichtung (70), wobei die Versiegelungseinrichtung (70) zum Versiegeln einer Öffnung (110) des Laborprobenbehälters (100) durch Anbringen eines Verschlusses (130) an dem Laborprobenbehälter (100) mit einem Haftmittel (140) ausgelegt ist, wobei eine Haftfestigkeit (AS1) des Haftmittels (140) durch Behandlung verringert werden kann (AS2), und
- eine Laborvorrichtung (20) nach Anspruch 8.

10. Laborautomationssystem (10) nach Anspruch 9, wobei die Versiegelungseinrichtung (70) Folgendes umfasst:
- mindestens eine Rollenaufnahme- und -bewegungseinrichtung (50), wobei die Rollenaufnahme- und -bewegungseinrichtung (50) zum Versehen des Verschlusses (130) mit einem Rollenband (150, 151) und zum Bewegen des Rollenbandes (150, 151) mit dem Verschluss (130) zu der Öffnung (110) des Laborprobenbehälters (100) zum Versiegeln ausgelegt ist.

## Revendications

1. Procédé de descellement d'une ouverture (110) d'un récipient d'échantillons de laboratoire (100) contenant un échantillon (120), dans lequel l'ouverture (110) est scellée par une fermeture (130) fixée au récipient d'échantillons de laboratoire (100) par un adhésif (140), dans lequel une résistance adhésive (AS1) de l'adhésif (140) peut être abaissée (AS2) par traitement par rayonnement, en particulier par rayonnement UV (RA), et/ou par traitement thermique, en particulier par de la chaleur (HE) avec une température (T) d'au minimum 50 °C, en particulier d'au minimum 120 °C, le procédé comprenant les étapes :
- a) traitement de l'adhésif (140), de sorte que sa résistance adhésive (AS1) est abaissée (AS2), l'étape a) comprenant : le traitement de l'adhésif (140) par rayonnement (RA), en particulier par rayonnement UV (RA), et/ou par de la chaleur (HE), en particulier par de la chaleur (HE) avec une température (T) d'au minimum 50 °C, en particulier d'au minimum 120 °C, et
- b) retrait de la fermeture (130) du récipient d'échantillons de laboratoire (100)
- le procédé étant **caractérisé en ce que** l'étape b) comprend :
la fixation d'un dispositif de prise (42) à la fermeture (130) par un autre adhésif (43), dans lequel une autre résistance adhésive (AS3) de l'autre adhésif (43) est telle qu'une autre force adhésive entre le dispositif de prise (42) et la fermeture fixée (130) est supérieure à une force adhésive entre la fermeture (130) et le récipient d'échantillons de laboratoire (100) par la résistance adhésive abaissée (AS2) de l'adhésif traité (140), et le retrait du dispositif de prise (42) avec la fermeture fixée (130) du récipient d'échantillons de laboratoire (100),
- l'étape b) comprenant en outre : le retrait de la fermeture (130) du récipient d'échantillons de laboratoire (100) par au moins un élément de réception et de déplacement de rouleau (60) fournissant le dispositif de prise (42) par une bande en rouleau (160) et déplaçant la bande en rouleau (160) avec le dispositif de prise (42) avec la fermeture fixée (130) loin du récipient d'échantillons de laboratoire (100).

2. Procédé selon la revendication 1, l'étape a) comprenant :
- l'exposition de l'adhésif (140) au rayonnement (RA) le long d'une direction (x) qui est différente d'une ligne (LI) entre l'adhésif (140) et l'échantillon (120) et/ou
- la focalisation du rayonnement (RA) sur l'adhésif (140).

3. Procédé selon la revendication 1 ou 2,
- dans lequel l'adhésif (140) comprend un site réactif au rayonnement UV (RA) et/ou un agent moussant.

4. Procédé de manipulation d'un récipient d'échantillons de laboratoire (100) contenant un échantillon (120), le procédé comprenant les étapes :
- scellement d'une ouverture (110) du récipient d'échantillons de laboratoire (100) par fixation d'une fermeture (130) sur le récipient d'échantillons de laboratoire (100) par un adhésif (140), dans lequel une résistance adhésive (AS1) de l'adhésif (140) peut être abaissée (AS2) par traitement, et
- descellement de l'ouverture (110) par un procédé selon l'une quelconque des revendications précédentes.

5. Procédé selon la revendication 4,
- dans lequel l'adhésif (140) est sensible à la pression, et
- le scellement comprenant : la pression de la fermeture (130) contre le récipient d'échantillons de laboratoire (100).

6. Procédé selon la revendication 4 ou 5, comprenant les étapes :
- fourniture de la fermeture (130) par une bande de rouleau (150, 151) et déplacement de la bande de rouleau (150, 151) avec la fermeture (130) jusqu'à l'ouverture (110) du récipient d'échantillons de laboratoire (100) pour le scellement.

7. Procédé selon l'une quelconque des revendications 4 à 6, le scellement comprenant :
- la fixation d'une ébauche de fermeture (151) au récipient d'échantillons de laboratoire (100) et
- la découpe de la fermeture (130) dans l'ébauche de fermeture (151).

8. Appareil de laboratoire (20) pour le descellement d'une ouverture (110) d'un récipient d'échantillons de laboratoire (100) contenant un échantillon (120), dans lequel l'ouverture (110) est scellée par une fermeture (130) fixée au récipient d'échantillons de laboratoire (100) par un adhésif (140), dans lequel une résistance adhésive (AS1) de l'adhésif (140) peut être abaissée (AS2) par traitement par rayonnement, en particulier par rayonnement UV (RA), et/ou par traitement thermique, en particulier par de la chaleur (HE) avec une température (T) d'au minimum 50 °C, en particulier d'au minimum 120 °C, l'appareil de laboratoire (20) comprenant :
- un dispositif de traitement (30), dans lequel le dispositif de traitement (30) est adapté pour traiter l'adhésif (140), de sorte que sa résistance adhésive est abaissée (AS2), dans lequel le dispositif de traitement (30) est adapté pour traiter l'adhésif (140) par rayonnement (RA), en particulier par rayonnement UV (RA), et/ou par de la chaleur (HE), en particulier par de la chaleur (HE) avec une température (T) d'au minimum 50 °C, en particulier d'au minimum 120 °C, et
- un dispositif de retrait (40), dans lequel le dispositif de retrait (40) est adapté pour retirer la fermeture (130) du récipient d'échantillons de laboratoire (100),
- **caractérisé en ce que** le dispositif de retrait (40) est adapté pour fixer un dispositif de prise (42) à la fermeture (130) par un autre adhésif (43), dans lequel une autre résistance adhésive (AS3) de l'autre adhésif (43) est telle qu'une force adhésive entre le dispositif de prise (42) et la fermeture fixée (130) est supérieure à une force adhésive entre la fermeture (130) et le récipient d'échantillons de laboratoire (100) par la résistance adhésive abaissée (AS2) de l'adhésif (140) traité, et pour retirer le dispositif de prise (42) avec la fermeture fixée (130) du récipient d'échantillons de laboratoire (100),
- le dispositif de retrait (40) comprenant : au moins un élément de réception et de déplacement de rouleau (60), dans lequel l'élément de réception et de déplacement de rouleau (60) est adapté pour fournir le dispositif de prise (42) par une bande de rouleau (160) et pour déplacer la bande de rouleau (160) avec le dispositif de prise (42) avec la fermeture fixée (130) loin du récipient d'échantillons de laboratoire (100).

9. Système d'automatisation de laboratoire (10) pour la manipulation d'un récipient d'échantillons de laboratoire (100) contenant un échantillon (120), le système d'automatisation de laboratoire (10) comprenant :
- un dispositif de scellement (70), dans lequel le dispositif de scellement (70) est adapté pour sceller une ouverture (110) du récipient d'échantillons de laboratoire (100) par fixation d'une fermeture (130) au récipient d'échantillons de laboratoire (100) par un adhésif (140), dans lequel une résistance adhésive (AS1) de l'adhésif (140) peut être abaissée (AS2) par traitement, et
- un appareil de laboratoire (20) selon la revendication 8.

10. Système d'automatisation de laboratoire (10) selon la revendication 9, le dispositif de scellement (70) comprenant :
- au moins un élément de réception et de déplacement de rouleau (50), dans lequel l'élément de réception et de déplacement de rouleau (50) est adapté pour fournir la fermeture (130) par une bande de rouleau (150, 151) et pour déplacer la bande de rouleau (150, 151) avec la fermeture (130) jusqu'à l'ouverture (110) du récipient d'échantillons de laboratoire (100) pour le scellement.
